# EUROPEAN PATENT APPLICATION

(11) **EP 2 006 368 A2**
(43) Date of publication of application: **24.12.2008**
(21) Application number: 07739940.0
(22) Date of filing: 27.03.2007
(51) Int. Cl.: C12M 1/00

(54) **TIP FOR CELL FUSION**

(30) Priority: 07.04.2006 JP 2006107001
(71) Applicant: ABsize Inc., Osaka-shi, Osaka 530-0053 (JP)
(72) Inventor: OH, Isamu, Suita-shi, Osaka 565-0871 (JP); MASUHARA, Hiroshi, Suita-shi, Osaka 565-0871 (JP); YAMATO, Toshiyuki, Ibaraki-shi, Osaka 567-0085 (JP); HAYASHI, Katsuaki, Ibaraki-shi, Osaka 567-0085 (JP); ADACHI, Yasuji, Ibaraki-shi, Osaka 567-0085 (JP); SATO, Setsuya, Ibaraki-shi, Osaka 567-0085 (JP)
(74) Representative: Skuhra, Udo
(86) International application number: PCT/JP2007/056502
(87) International publication number: WO 2007/116771

(57) **Abstract**

To provide a cell fusion chip, which allows to perform a cell fusion on a single chip, excels in the operating efficiency, does not require experience so much in cell feed or recovery, and has no risk of the contamination to the cells during the operation.

(Means for solving problems)

The present invention provides a cell fusion chip comprising: an isolation chamber for receiving isolated cells to be subjected to an fusion operation; a fusion chamber for fusing the cells together supplied from the isolation chamber; a culture chamber for culturing the cells fused in the fusion chamber; a first channel for connecting the isolation chamber and the fusion chamber; and a second channel for connecting the fusion chamber and the culture chamber, wherein the isolation chamber, the fusion chamber, the culture chamber, the first channel and the second channel are formed on a single chip.

## Description

### FIELD OF THE INVENTION

The present invention relates to a cell fusion chip, and more specifically, which excels in operating a cell fusion on a single chip efficiently.

### DESCRIPTION OF THE RELATED ART

Generally, a cell fusion operation is carried out under microscopic observation for cells in suspension received in a vessel placed on a stage of a microscope. (For example, see Patent document 1.)

In such a cell fusion operation, typically manipulation tools such as a micropipette is used to supply cells to the vessel on the microscope stage or recover the fused cells from/to the other vessel.

However, according to the above-mentioned operation, the feed or the recovery of the cells requires a lot of experience and gives an opportunity of contamination on the cells during their transportation. Also the method requiring multiple vessels results in lower efficiency.

[Patent document 1]Japanese patent publication 4-349889

### DISCLOSURE OF THE INVENTION PROBLEMS TO BE SOLVED BY THE INVENTION

The objective of the present invention is to solve the above problems of the related art. The present invention provides a cell fusion chip, which allows to perform a cell fusion on a single chip, excels in the operating efficiency, does not require experience so much in cell feed or recovery, and has no risk of the contamination to the cells during the operation.

### MEANS FOR SOLVING THE PROBLEMS

The present invention according to claim 1 is related to a cell fusion chip comprising:an isolation chamber for receiving isolated cells to be subjected to an fusion operation; a fusion chamber for fusing the cells together supplied from the isolation chamber; a culture chamber for culturing the cells fused in the fusion chamber; a first channel for connecting the isolation chamber and the fusion chamber; and a second channel for connecting the fusion chamber and the culture chamber, wherein the isolation chamber, the fusion chamber, the culture chamber, the first channel and the second channel are formed on a single chip.

The present invention according to claim 2 is related to the cell fusion chip according to claim 1, comprising: a connection port for connecting to a microsyringe pump; and a third channel for connecting to the connection port; wherein at least one of the isolation chamber, the fusion chamber and the culture chamber is connected to the connection port through the third channel.

The present invention according to claim 3 is related to the cell fusion chip according to claim 2, wherein the third channel connected to the fusion chamber comprises one channel connected to one connection port and another channel connected to another connection port.

The present invention according to claim 4 is related to the cell fusion chip according to any of claims 1 to 3, wherein the fusion chamber is wider than the first to third channels.

The present invention according to claim 5 is related to the cell fusion chip according to any of claims 1 to 4, wherein a section containing the culture chamber is formed to be separable from a section containing the other chambers.

The present invention according to claim 6 is related to the cell fusion chip according to any of claims 1 to 5, wherein the fusion chamber comprises a couple of electrodes disposed oppositely each other.

The present invention according to claim 7 is related to the cell fusion chip according to any of claims 1 to 6, further comprising a wall with a concave in the fusion chamber.

The present invention according to claim 8 is related to the cell fusion chip according to any of claims 1 to 7, further comprising a wall grid-patterned in a planar view in the fusion chamber.

The present invention according to claim 9 is related to the cell fusion chip according to claim 7, wherein the wall comprises a couple of walls placed between the opposite electrodes so that their concaves are aligned oppositely with respect to each other.

The present invention according to claim 10 is related to the cell fusion chip according to claim 8, wherein the wall is placed between the opposite electrodes.

### EFFECT OF THE INVENTION

The present invention described in the claim 1 allows to receive isolated cells, electrically fuse the cells and culture the fused cells in the separate chambers on a single chip. Thus, it does not require experience so much in cell feed or recovery, and has no risk of the contamination to the cells during the operation. This results in improving the operating efficiency.

The present invention described in the claim 2 allows to transfer the cells between the chambers with the microsyringe pump. Thus, the cells are transferred easily and certainly, furthermore, quickly and precisely.

The present invention described in the claim 3 allows to directly supply a culture medium to the fusion chamber and remove the culture medium from the fusion chamber without its crossing over the other chambers. In addition, such operations are performed easily and precisely with the microsyringe pump.

The present invention described in the claim 4 allows to certainly prevent an unintended cell from in-flow from the isolation chamber to the fusion chamber or out-flow from the fusion chamber to the culture chamber.

According to the present invention described in the claim 5, the separated section containing the culture chamber from the section containing the other chamber after transferring the fused cells into the culture chamber independently works as a culture chamber member.

According to the present invention described in the claim 6, the fusion chamber comprises a couple of electrodes disposed oppositely each other. The application of an AC voltage between the opposite electrodes allows to easily align the cells each other to form a pearl chain. This results in enhancing efficiency of the cell fusion manipulation.

According to the present invention described in the claim 7, the cell fusion chip further comprises a wall with a concave in the fusion chamber. Thus, the concave facilitates the compression between cells each other when they are pushed into the concave to increase a cell fusion rate because of an enhanced adherence between them.

According to the present invention described in the claim 8, the cell fusion chip further comprises a wall grid-patterned in a planar view in the fusion chamber. Thus, the movement of the cells can be restricted by pushing the cells into the grid. This results in easy manipulation for the cell fusion. Further, it is possible to carry out the cell fusion manipulation with a selectivity using laser tweezers to control the number of cells to be pushed into the grid. In addition, the cell separation by the grid makes it easy to remove the fused cells using an external manipulator.

According to the present invention described in the claim 9, the wall comprises a couple of walls placed between the opposite electrodes so that their concaves are aligned oppositely with respect to each other. The concave immobilizes a pearl chain formed by application of AC voltage between the opposite electrodes to facilitate targeting the cells for cell fusion with a UV laser and the like.

According to the present invention described in the claim 10, the wall is placed between the opposite electrodes. The grid immobilizes a pearl chain formed by application of AC voltage between the opposite electrodes to facilitate targeting the cells for the cell fusion with a UV laser and the like.

### DESCRIPTION OF THE PREFERRED EMBODIMENTS

Hereinafter, preferred embodiments of the cell fusion chip according to the present invention will be explained with reference to the drawings.

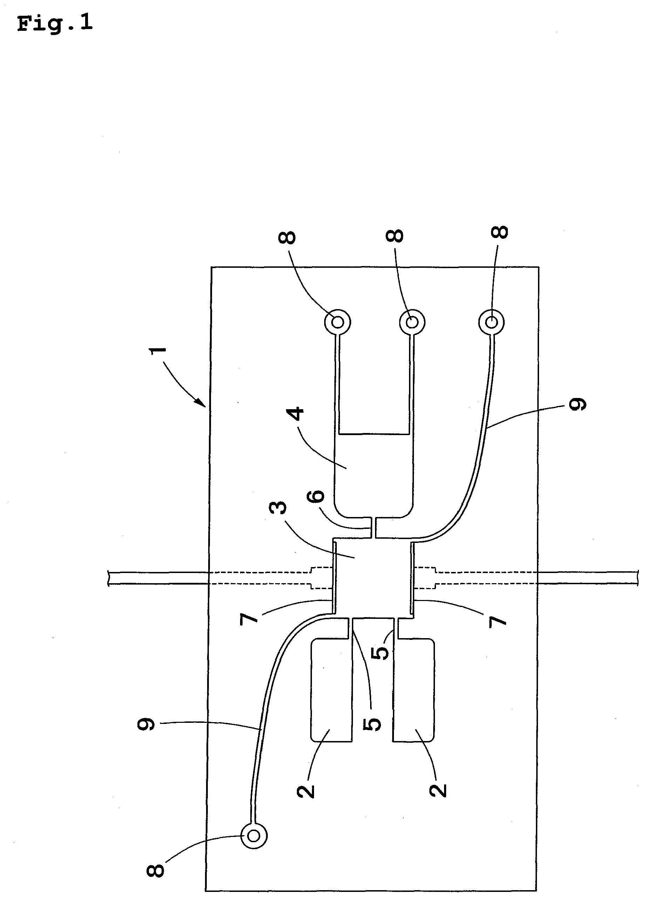

Fig.1 is a plain view of a cell fusion chip according to the present invention.
A cell fusion chip (1) of the present invention is made of a piece of a flat plate comprising an insulating material (for example, synthetic resin such as PP, PE, PET and the like). Multiple depressed chambers are on the flat plate.
The chamber comprises three kinds of chambers which are an isolation chamber (2), a fusion chamber (3) and a culture chamber (4). These three kinds of chambers are formed on a single chip.

The isolation chamber (2) receives a suspension including isolated cells to be subjected to a fusion manipulation.
The cell fusion manipulation among an identical type of cells requires just one isolation chamber (2) while the manipulation among different types of cells requires multiple isolation chambers (2) for each type of cells to be placed separately. In the case of the multiple isolation chambers (2), the number of chambers is not limited to, but may be two (as shown), three or more. In addition, a size of each chamber may be the same or may be different.

The fusion chamber (3) is used for fusion manipulation to cells supplied from the isolation chamber (3).
On the inner wall of the fusion chamber (3), a couple of electrodes (7) are disposed oppositely each other with a certain distance.
A couple of electrodes (7) are respectively connected to an electric power source (not shown) so that an AC voltage can be applied between the opposite electrodes.

As for each electrodes (7), one end is bare in the inner wall of the isolation chamber (2), the other end is bare in the outside of the chip (1), and the remaining part is buried into the insulating material constituting the cell fusion chip (1) in order to prevent a conduction except the inside of the isolation chamber (2).

The culture chamber (4) is used for receiving and culturing the cells fused in the fusion chamber (3).
Two or more chambers may be formed to separately culture the cells in each culture chamber (4), although the number of culture chambers (4) is only one in the figure shown.

On the upper surface of the cell fusion chip (1), multiple channels are formed for communication among the above three kinds of chambers (2), (3) and (4). Through these multiple channels, a fluid (such as liquid containing cells) may be mutually distributed among these different kinds of the chambers.

A first channel (5) connects the isolation chamber (2) to the fusion chamber (3). The cells in the isolation chamber (2) are transferred to the fusion chamber (3) through the first channel (5) to be subjected to the fusion manipulation therein.

A second channel (6) connects the fusion chamber (3) to the culture chamber (4). The fused cells in the fusion chamber (3) are transferred to the culture chamber (4) through the second channel (6) to be cultured in the culture chamber (4).

The upper surface of the cell fusion chip (1) comprises a connection port (8) configured to be connected to discharge/suction ports of a microsyringe pump.
The connection port (8) is connected to at least one of the isolation chamber (2), the fusion chamber (3) and the culture chamber (4) through a third channel (9).
This structure enables to introduce a liquid including a culture medium or cells into each chamber or remove the liquid from each chamber while precisely controlling the flow rate with the microsyringe pump.

In the figure shown, two kinds of chamber, the fusion chamber (3) and the culture chamber (4), are connected to the connection ports (8) through the third channels (9).

The third channels (9) configured to connect to the fusion chamber (3) are extended in an area where the isolation chamber (2) is located and in an area where the culture chamber (4) is located respectively in order to be connected with the separate connection ports (8).
The connection port (8) at the end of the third channel (9) running beside the isolation chamber (2) is configured to introduce the culture medium into the fusion chamber (3) with the microsyringe pump while the connection port (8) at the end of the third channel (9) running beside the culture chamber (3) is configured to remove the culture medium from the fusion chamber (3) with the microsyringe pump.
This structure enables to directly supply the culture medium to the fusion chamber (3) and remove the culture medium from the fusion chamber (3) without its crossing over the other chambers with the microsyringe pump.

The third channels (9) configured to be connected with the culture chamber (4) are extended in a direction to be apart from the fusion chamber (3).
The connection ports (8) at the ends of the third channels (9) are configured to remove the culture medium from the culture chamber (4) with the microsyringe pump.

In addition, the third channel (9) may be also extended from the isolation chamber (2) to be connected with the connection port (8) of the microsyringe pump in the present invention (not shown).

In the present invention, the isolation chamber (2), the fusion chamber (3) and the culture chamber (4) are sufficiently wider than the above-described first to third channels (5), (6) and (9) (for example, twice or more, more preferably 5 times or more).
This structure enables to certainly prevent an unintended cell from in-flow from the isolation chamber (2) to the fusion chamber (3) or out-flow from the fusion (3) chamber to the culture chamber (4).

Preferable placement of a set of the first channel (5) and the second channel (6), the second channel (6) and the third channel (7) or the first channel (5) and the third channel (7) may not in an alignment manner as shown in Fig. 1.
This structure also excels in preventing an unintended cell from in-flow from the isolation chamber (2) to the fusion chamber (3) or out-flow from the fusion chamber (3) to the culture chamber (4).

Further, in the cell fusion chip (1) of the present invention, a section containing the culture chamber (4) is preferably formed to be separable from a section containing the other chambers (2) and (3) in order to independently use the separated section as a culture chamber member.

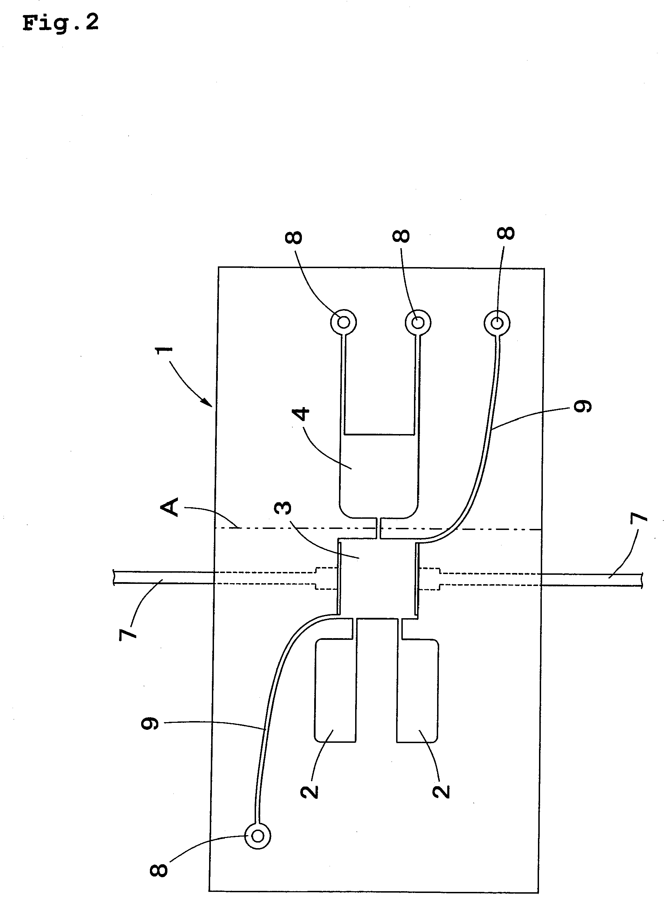

Fig.2 is a plain view of a cell fusion chip with the above structure. In the figure, a line shown as a hidden line (A) is a border line to be separated. The cell fusion chip (1) is divided into the section containing the culture chamber (4) and the section containing the other chambers (2) and (3) by separating the cell fusion chip (1) along the border line (A).

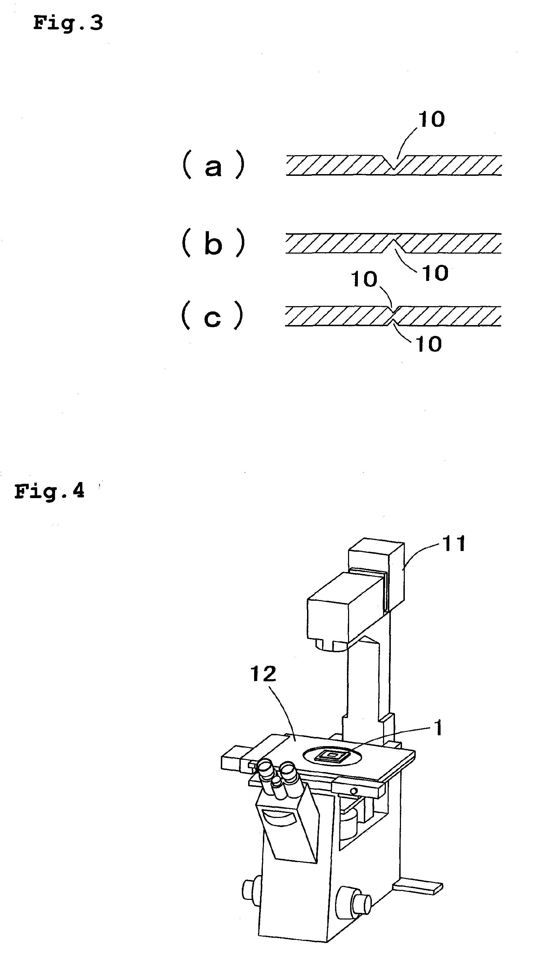

The border line (A) for separating the cell fusion chip (1) may comprise, for example, a groove (10) formed in the chip (1) as shown in Fig. 3.
Fig. 3 (a) shows that the groove (10) is formed on the top surface of the chip (1), Fig. 3 (b) shows that the groove (10) is formed on the bottom surface of the chip (1), and Fig. 3 (c) shows that the grooves (10) are formed on the both top and bottom surfaces of the chip (1), respectively. Each groove is formed with an acute angle in a cross-section.
Such a groove (10) allows to easily divide the chip (1) along the groove (10).

In addition, the structure for separating the cell fusion chip (1) is not limited to the groove (10).
For example, the cell fusion chip (1) may be preliminarily formed from two pieces which are the section containing the culture chamber (4) and the section containing the other chambers (2) and (3). When the cell fusion is performed, these two pieces are combined together to form a single chip (1) by a coupling means capable of reseparating the two pieces if desired, such as a fitting, pinning and the like. Further, the chip (1) may be divided into the two pieces again after the fusion manipulation.

In addition, after the section containing the culture chamber (4) is separated from the section containing the other chambers (2) and (3), the end (separated side) of the second channel (6) is preferably blocked with an optional sealing means (ex. adhesive and the like) in order to prevent the liquid in the culture chamber (4) from leaking in the second channel (6).

The cell fusion chip (1) comprising the above structure according to the present invention is placed and used on the electrical stage (12) of a microscope (11) as shown in Fig.4.
The microscope (11) comprises laser sources for a laser trapping to capture and operate cells and for a cell fusion to fuse cells by laser (not shown).
As for the trapping laser, for example, IR laser such as YAG laser (wavelength 1060nm), Nd:YLF laser (wavelength 1047nm), DPSS laser (wavelength 1064nm) and the like is used. As for the laser for a cell fusion, for example, UV laser is used.

In addition to the above first and second laser sources, the microscope (11) may further comprise a third laser source for outputting an ultrashort pulse laser (picosecond laser or femtosecond laser).
The ultrashort pulse laser is preferably used as the trapping laser when the cell manipulation requires a strong force.

In addition to the above laser manipulators, the microscope (11) may further comprise a mechanical manipulator such as a micropipette and the like equipped with a thin metal needle or a glass tube on the electrical stage (12).

The microscope (11) comprising the laser source or the mechanical manipulator as described-above allows to provide the trapping and the cell fusion by the laser or the operation by the mechanical manipulator to the cells contained in the cell fusion chip (1) on the electrical stage (12).

The cell fusion manipulation by using the cell fusion chip (1) of the present invention is carried out on the electrical stage (12) of the microscope (11) according to the following procedures.
At first, a suspension containing isolated cells to be subjected to a fusion manipulation is supplied to the isolation chamber (2) in the cell fusion chip (1).
Next, the suspension supplied to the isolation chamber (2) is transferred to the fusion chamber (3) through the first channel (5) by using the manipulator (laser or mechanical type) equipped with the microscope (11) or the microsyringe pump.

When the suspension containing the cells is received in the fusion chamber (3), an AC voltage is applied between a couple of electrodes (7) placed in the fusion chamber (3).
When the AC voltage is applied thereto, the cells in the suspension between electrodes are aligned parallel to the direction of the electrical field to form a pearl chain. Then, by irradiating the aligned cells with a laser for the cell fusion, the cells are fused together.

The fused cells are transferred to the culture chamber (4) through the second channel (6) by using the manipulator (laser or mechanical type) equipped with the microscope (11) or the microsyringe pump.

When the fused cells are received in the culture chamber (4), the cell fusion chip (1) is removed from the electrical stage (12) of the microscope (11) to culture the cells in the culture chamber (4).
At this time, the section containing the culture chamber (4) is separated from the section containing the other chambers (2) (3). Thus, the separated section can be independently used as a culture chamber member.

As described-above, the cell fusion chip (1) according to the present invention allows to receive isolated cells, electrically fuse the cells and culture the fused cells on a single chip.
Thus, the cell fusion chip (1) does not require a skill in supplying or taking out the cells, which has been necessary in the conventional cell fusion methods. Further, it has no risk of the contamination to the cell during the operation. This results in improving the operating efficiency of the cell fusion manipulation.

In the cell fusion chip (1) of the present invention, the structure of the fusion chamber (3) may be changed as below.

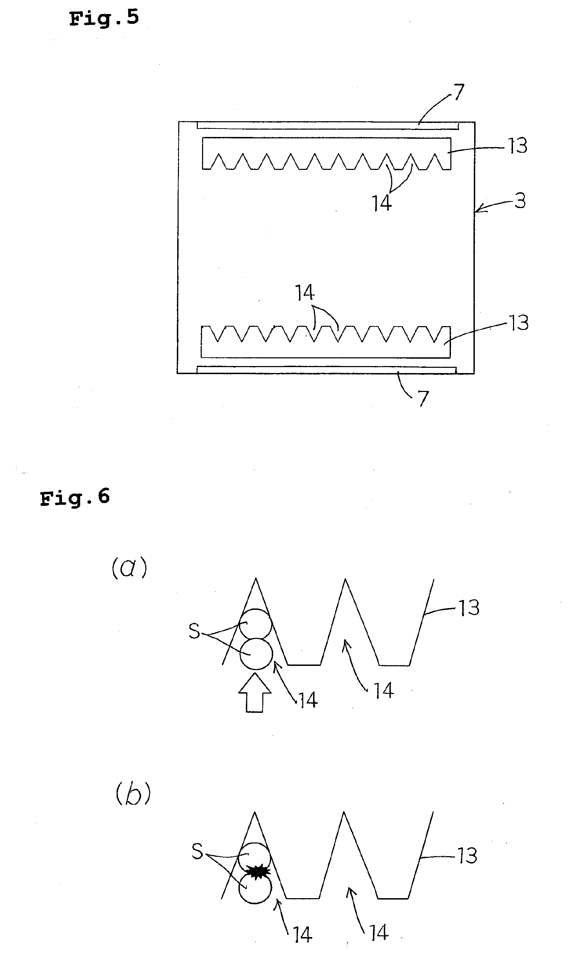

Fig.5 shows a first alternative embodiment of the fusion chamber (3) in the cell fusion chip (1) according to the present invention. Here, the structure except the fusion chamber (3) is the same as Fig.1, and channels are omitted and not shown.
The first alternative embodiment differs in that a wall (13) with a concave (14) is comprised in the fusion chamber (3), and the other structures are the same as Fig.1.

In the figure shown, the wall (13) with the concave (14) comprises a couple of walls placed between the opposite electrodes (7) so that their concaves (14) are aligned oppositely with respect to each other.
The number of concaves (14) placed in the wall (13) is not limited to, but multiple concaves (14) are preferably aligned and placed as shown in the figure. The shape of the concave (14) is also not limited to, but a triangular concave (14) in a planar view inwardly expanded is preferably used as shown in the figure because the cells are well fitted and fixed to the concave (14). The size of the concave (14) has preferably the width and depth which allow multiple cells to enter and align into the concave (14) to form just a single line.

In the cell fusion manipulation using the cell fusion chip comprising the fusion chamber of the first alternative embodiment, at first, multiple cells (S) are fitted and fixed to the concave (14) by using laser tweezers or applying an AC voltage to the opposite electrodes as shown in Fig. 6(a). Subsequently, the cells are fused together by using a UV laser or applying a DC voltage as shown in Fig. 6(b).

As shown above, according to the cell fusion manipulation using the cell fusion chip comprising the fusion chamber of the first alternative embodiment, the cells are strongly pushed each other by fitting the cells into the concave. This results in improving an adherence property of cells and a cell fusion rate.
In addition, the concave immobilizes a pearl chain formed by application of AC voltage between the opposite electrodes to facilitate targeting the cells for cell fusion with a UV laser and the like.

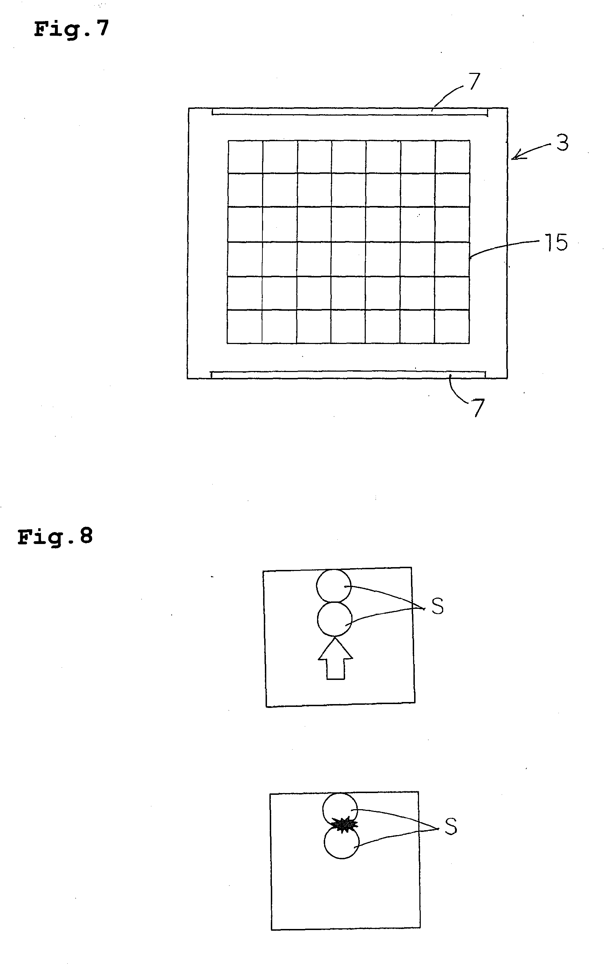

Fig. 7 shows a second alternative embodiment of the fusion chamber (3) in the cell fusion chip (1) according to the present invention. Here, the structure except the fusion chamber (3) is the same as Fig.1, and channels are omitted and not shown.
The second alternative embodiment differs in that a wall (15) grid-patterned in a planar view is comprised in the fusion chamber (3), and the other structures are the same as Fig.1.

In the figure shown, the grid-patterned wall (15) is placed between the opposite electrodes (7).
The size of each grid (square) is set depending on the number of cells to be fused, and it has to contain at least 2 cells. In addition, the shape of each grid may be rectangular, triangular, hexagonal, as well as square. The height of the wall forming the grid is set slightly higher than the cell.

In the cell fusion manipulation using the cell fusion chip comprising the fusion chamber of the second alternative embodiment, multiple cells (S) are introduced into the grid with laser tweezers or the like and aligned in the grid by applying an AC voltage to the opposite electrodes (7) as shown in Fig. 8 (a). After that, the cells are fused together by using a UV laser or applying a DC voltage, as shown in Fig. 8 (b). Here, a single square of the grid is shown in Fig.8.

As shown above, according to the cell fusion manipulation using the cell fusion chip comprising the fusion chamber of the second alternative embodiment, the cells are fitted into the grid to restrict their movement. This results in easily manipulating the cell fusion. Further, it is possible to carry out the cell fusion manipulation with a selectivity using laser tweezers to control the number of cells to be pushed into the grid. In addition, the cell separation by the grid makes it easy to remove the fused cells using an external manipulator.

In the cell fusion chip comprising the fusion chamber of the first and the second alternative embodiments, the cell fusion chip without the opposite electrodes (7) in the fusion chamber (3) has a certain effect. Thus, the cell fusion chip which does not comprise the opposite electrodes (7) in the fusion chamber (3) may be also used.

### INDUSTRIAL APPLICABILITY

The present invention is highly available in biotechnology study centering on new varieties of animals and plants and regenerative medicines, such as an establishment of an artificial insemination on high plants, a functional activity analysis of a cell network, a search of cell-affecting proteins, chemicals and the like, and a development of a patterning method for a novel cell.

### BRIEF DESCRIPTION OF THE DRAWINGS

Fig.1 is a plain view of a cell fusion chip according to the present invention.
Fig.2 is a plain view of a cell fusion chip according to the present invention when a section containing a culture chamber is formed to be separable from a section containing other chambers.
Fig.3 shows that a groove is formed with an acute angle in a cross-section on the chip as a border line for separating the cell fusion chip.
Fig.4 shows a use situation of the cell fusion chip according to the present invention.
Fig.5 shows a first alternative embodiment of the cell fusion chamber in the cell fusion chip according to the present invention.
Fig.6 shows an effect of the cell fusion chamber of the first alternative embodiment.
Fig.7 is a plain view of the second alternative embodiment of the cell fusion chamber in the cell fusion chip according to the present invention.
Fig.8 shows an effect of the cell fusion chamber of the second alternative embodiment.

### EXPLANATION OF REFERENCE NUMERALS

- 1.: cell fusion chip
- 2.: isolation chamber
- 3.: fusion chamber
- 4.: culture chamber
- 5.: first channel
- 6.: second channel
- 7.: electrode
- 8.: connection port
- 9.: third channel
- 10.: groove
- 11.: microscope
- 12.: electrical stage
- 13.: wall with a concave
- 14.: concave
- 15.: wall grid-patterned in a planar view
- S: cell

## Claims

1. A cell fusion chip comprising:
an isolation chamber for receiving isolated cells to be subjected to an fusion operation;
a fusion chamber for fusing the cells together supplied from the isolation chamber;
a culture chamber for culturing the cells fused in the fusion chamber;
a first channel for connecting the isolation chamber and the fusion chamber; and
a second channel for connecting the fusion chamber and the culture chamber,
wherein the isolation chamber, the fusion chamber, the culture chamber, the first channel and the second channel are formed on a single chip.

2. The cell fusion chip according to claim 1, comprising:
a connection port for connecting to a microsyringe pump; and
a third channel for connecting to the connection port;
wherein at least one of the isolation chamber, the fusion chamber and the culture chamber is connected to the connection port through the third channel.

3. The cell fusion chip according to claim 2,
wherein the third channel connected to the fusion chamber comprises one channel connected to one connection port and another channel connected to another connection port.

4. The cell fusion chip according to any of claims 1 to 3,
wherein the fusion chamber is wider than the first to third channels.

5. The cell fusion chip according to any of claims 1 to 4,
wherein a section containing the culture chamber is formed to be separable from a section containing the other chambers.

6. The cell fusion chip according to any of claims 1 to 5,
wherein the fusion chamber comprises a couple of electrodes disposed oppositely each other.

7. The cell fusion chip according to any of claims 1 to 6, further comprising a wall with a concave in the fusion chamber.

8. The cell fusion chip according to any of claims 1 to 7, further comprising a wall grid-patterned in a planar view in the fusion chamber.

9. The cell fusion chip according to claim 7,
wherein the wall comprises a couple of walls placed between the opposite electrodes so that their concaves are aligned oppositely with respect to each other.

10. The cell fusion chip according to claim 8,
wherein the wall is placed between the opposite electrodes.
